(19) Europäisches Patentamt
European Patent Office
Office européen des brevets

(11) Veröffentlichungsnummer : **0 612 994 A2**

(12) **EUROPÄISCHE PATENTANMELDUNG**

(21) Anmeldenummer : **94810091.2**

(22) Anmeldetag : **17.02.94**

(51) Int. Cl.[5] : **G01N 1/28,** H01J 49/16, C12Q 1/68

(30) Priorität : **26.02.93 CH 591/93**
**23.07.93 CH 2233/93**

(43) Veröffentlichungstag der Anmeldung :
**31.08.94 Patentblatt 94/35**

(84) Benannte Vertragsstaaten :
**CH DE FR GB IT LI SE**

(71) Anmelder : **CIBA-GEIGY AG**
**Klybeckstrasse 141**
**CH-4002 Basel (CH)**

(72) Erfinder : **Pieles, Uwe, Dr.**
**St. Michaelstrasse 27**
**D-79189 Bad Krozingen (DE)**

(54) **Matrix für die matrix-unterstützte Laserdesorptions-Massenspektroskopie.**

(57) Die Erfindung betrifft eine Komposition enthaltend
(a) mindestens ein Ammoniumsalz einer anorganischen oder organischen Säure,
(b) eine Verbindung der Formel I,

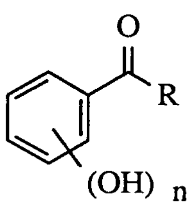

(I)

worin R einen aliphatischen Kohlenwasserstoff-Rest mit 1 bis 12 C-Atomen oder einen mit -O- Atomen unterbrochenen aliphatischen Kohlenwasserstoff-Rest mit 2 bis 12 C-Atomen bedeutet und n eine Zahl von 1 bis 5 bedeutet und
(c) mindestens eine höhermolekulare Verbindung aus der Gruppe der Polynukleotide, Oligosaccharide, Proteine und makrozyklischen Metallkomplexe,
sowie deren Anwendung in der matrix-unterstützten Laserdesorptions-Massenspektro- skopie von Oligonukleotidsequenzen.

EP 0 612 994 A2

Die Erfindung betrifft eine Komposition, welche ein im Phenylteil substituiertes Phenon, ein Ammoniumsalz und eine höhermolekulare Verbindung enthält, sowie deren Anwendung in der matrix-unterstützten Laserdesorptions-Massenspektroskopie von Oligonukleotidsequenzen.

Die matrix-unterstützte Laserdesorptions-Massenspektroskopie gestattet es, Molekulargewichte von Peptiden und Proteinen von wenigen hundert bis zu mehr als 100 000 Dalton zu bestimmen. Dabei spielt die Matrix eine wesentliche Rolle, in welche das Protein eingebettet wird. In der DE-A-40 17 804 und in der WO 91/04570 werden Verbindungen beschrieben, welche als Matrizes in der Laserdesorptions-Massenspektroskopie von Biomolekülen Verwendung finden. Vergleichbare Versuche der Molekulargewichtsbestimmung von Oligonukleotiden waren bisher nur wenig erfolgreich und sind z.B. bei G.R. Parr et al., Rapid Communications in Mass Spectrometry, Vol. 6, Seiten 369 bis 372 (1992) und K. Tang et al., Rapid Communications in Mass Spectrometry, Vol. 6, Seiten 365 bis 368 (1992) beschrieben. Bei den dort beschriebenen Methoden beobachtet man, sofern überhaupt eine Laserdesorption erreicht werden kann, im Fall kleiner Oligonukleotide mehrere Massen-Peaks, die durch die Bildung von Addukten mit $Na^+$ und $K^+$ erklärt werden können und im Fall grösserer Oligonukleotide breite Peaks, die nur mehr eine wenig genaue Massenbestimmung zulassen. Es besteht daher ein grosses Interesse an neuen Matrizes zur Laserdesorption von Oligonukleotidionen.

Ein Gegenstand der Erfindung ist eine Komposition enthaltend

(a) mindestens ein Ammoniumsalz einer anorganischen oder organischen Säure

(b) eine Verbindung der Formel I,

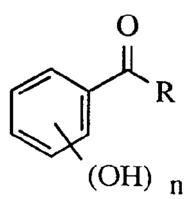

(I)

worin R einen aliphatischen Kohlenwasserstoff-Rest mit 1 bis 12 C-Atomen oder einen mit -O- Atomen unterbrochenen aliphatischen Kohlenwasserstoff-Rest mit 2 bis 12 C-Atomen bedeutet und n eine Zahl von 1 bis 5 bedeutet und

(c) mindestens eine höhermolekulare Verbindung aus der Gruppe der Polynukleotide, Oligosaccharide, Proteine und makrozyklischen Metallkomplexe.

Die Verbindung der Formel I und das Ammoniumsalz liegen bevorzugt in einem molaren Verhältnis von 1:5 bis 1:20 und besonders bevorzugt 1:8 bis 1:12 vor. Die höhermolekulare Verbindung und die Verbindung der Formel I werden bevorzugt in einem molaren Verhältnis von $1:5x10^3$ bis $1:5x10^5$, besonders bevorzugt $1:10^4$ bis $1:10^5$ eingesetzt.

Bei den verwendeten höhermolekularen Verbindungen kann es sich zum Beispiel um Poly- und Oligonukleotide des RNA- und DNA-Types handeln, um Oligosaccharide bestehend aus bevorzugt 2-10 glykosidisch verknüpften Zuckereinheiten, um Peptide und Proteine, die aus natürlich vorkommenden und/oder synthetisch hergestellten Aminosäuren bestehen oder um Metallkomplexe mit makrozyklischen, Heteroatomeenthaltenden Liganden wie z.B. Kronenether und $Na^+$-Ionophor Nonactin. Besonders bevorzugt verwendet werden Poly- und Oligonukleotide, die aus natürlichen oder synthetischen Nukleosidresten oder Mischungen solcher Nukleosidreste bestehen. Die verwendeten Oligonukleotide können dabei aus 2 bis 200 Nukleosidresten aufgebaut sein, bevorzugt jedoch aus 2 bis 100 und besonders bevorzugt aus 2 bis 60, insbesondere 2 bis 40. Dabei spielt es keine Rolle, ob es sich um natürlich vorkommende oder synthethisch modifizierte Nukleosidreste handelt. Im Fall der natürlichen Nukleosidreste kann es sich um Adenosin, Guanosin, Cytidin, Uridin, 2'-Desoxyadenosin, 2'Desoxythymidin, 2'-Desoxyguanosin und 2'-Desoxycytidin handeln. Bei den modifizierten Nukleosidresten sind besonders jene Nukleosidreste hervorzuheben, die sich von Adenin, N-Methyladenin, 2-Aminoadenin, 6-Hydroxypurin, 2-Amino-6-Chlorpurin, 2-Amino-6-methylthiopurin, Guanin, 7-Deazaguanin, N-Isobutyrylguanin, Uracil, Thymin, Cytosin, 5-Fluoruracil, 5-Chloruracil, 5-Bromuracil, Dihydrouracil und 5-Methylcytosin ableiten, sowie deren Phosphonate, Phosphorthioate, Phosphoramidate, Dephosphoderivate und carbocyclische Derivate. Synthetische Nukleosidreste sind in grosser Vielfalt bekannt und zum Beispiel in E. Uhlmann et al, Chemical Reviews, Vol. 90, Seiten 543-584 (1990); V. Marquez et al, Medicinal Research Reviews, Vol. 6, Seiten 1-40 (1986); und U. Englisch et al. Angewandte Chemie, Heft 6, Seiten 629-739 (1991) beschrieben.

In den durch die Formel I beschriebenen Verbindungen kann R lineares oder verzweigtes $C_1$-$C_{12}$-Alkyl, $C_1$-$C_{12}$-Hydroxyalkyl, $C_5$-$C_7$-Cycloalkyl, $C_5$-$C_7$-Hydroxycycloalkyl, mit -O- Atomen unterbrochenes lineares

oder verzweigtes $C_2$-$C_{12}$-Alkyl beziehungsweise $C_2$-$C_{12}$-Hydroxyalkyl, mit -O- Atomen unterbrochenes $C_5$-$C_7$-Cycloalkyl beziehungsweise $C_5$-$C_7$-Hydroxycycloalkyl oder Wasserstoff bedeuten. Bevorzugte Verbindungen der Formel I sind solche, bei denen R die Bedeutung von linearem oder verzweigtem Alkyl oder Cycloalkyl hat, besonders bevorzugt sind lineares oder verzweigtes $C_1$-$C_4$-Alkyl. 2,4,6-Trihydroxyacetophenon, 2,4,5-Trihydroxybutyrophenon, 2,4-, 2,5- und 2,6-Dihydroxyacetophenon können als typische Beispiele für Verbindungen der Formel I genannt werden.

Als wesentlicher Bestandteil der erfindungsgemässen Komposition erweist sich überraschenderweise die Anwesenheit eines Ammoniumsalzes einer anorganischen oder organischen Säure. Dabei kann es sich um die Ammoniumsalze der Halogenwasserstoffsäuren, der Perchlorsäure, der Chalkogensäuren, der Salpetersäure, der salpetrigen Säure, der Kohlensäure und der Borsäure, der Hydroxypolycarbonsäuren und der Säuren HOOC-$R_1$-COOH, worin $R_1$ für $C_1$-$C_8$-Alkylen, $C_5$-$C_6$-Cycloalkylen, Phenylen oder Naphthylen mit 1 bis 3 Hydroxygruppen steht, handeln. Kombiniert mit Oligonukleotiden und einer Verbindung der Formel I ermöglichen sie nicht nur die Laserdesorption von Oligonukleotidionen unabhängig von deren Basensequenz, sondern sie verhindern gleichzeitig die Bildung von Addukten mit z.B. $Na^+$ und $K^+$. Dies führt bei einer Verwendung der erfindungsgemässen Komposition in der matrix-unterstützten Laserdesorptions-Massenspektrometrie zu scharfen Molekül-Peaks der Oligonukleotidionen, deren entsprechende Molekülmasse exakt bestimmt werden kann. Bevorzugt eingesetzt werden die Salze der Schwefelsäure, Salpetersäure, Tartronsäure, Aepfelsäure, Weinsäure, Zitronensäure sowie der Säuren HOOC-$R_1$-COOH, worin $R_1$ für $C_1$-$C_8$-Alkylen, $C_5$-$C_6$-Cycloalkylen, Phenylen oder Naphthylen mit 1 bis 3 Hydroxygruppen steht. Darunter sind die Ammoniumsalze der Schwefelsäure, Weinsäure und Zitronensäure besonders bevorzugt.

Eine im Sinne der Erfindung typische Komposition ist schwach sauer mit einem pH-Wert um 6 und enthält mindestens ein Ammoniumsalz beispielsweise der Schwefelsäure, Weinsäure oder Zitronensäure, als Verbindung der Formel I beispielsweise 2,4,6-Trihydroxyacetophenon, 2,4,5-Trihydroxybutyrophenon, 2,4-, 2,5- oder 2,6-Dihydroxyacetophenon sowie mindestens ein Oligonukleotid bestehend aus natürlichen oder synthetischen Nukleosidresten oder Mischungen dieser Nukleosidreste.

Von besonderer Bedeutung ist die Verwendung der erfindungsgemässen Komposition in der matrix-unterstützten Laserdesorptions-Massenspektroskopie von Oligonukleotiden.

Ein weiterer Gegenstand der Erfindung ist ein Mittel zur Verwendung als Matrix in der Laserdesorption von Analytmolekülionen, welches mindestens ein Ammoniumsalz einer anorganischen oder organischen Säure und eine Verbindung der Formel I,

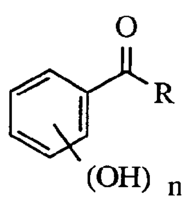

(I)

enthält, worin R und n die zuvor genannten Bedeutungen haben. Dabei beträgt das Verhältnis von Ammoniumsalz und Verbindung der Formel I bevorzugt 1:5 bis 1:20, besonders bevorzugt jedoch 1:8 bis 1:12.

Die Erfindung beinhaltet auch ein Verfahren zur Bestimmung der Molekulargewichte von Oligonukleotiden mit 2 bis 200, bevorzugt 2 bis 100 und besonders bevorzugt 2 bis 60, insbesondere 2 bis 40 Nukleosidresten mit Hilfe der matrix-unterstützten Laserdesorptions-Massenspektroskopie, worin das zuvor beschriebene und erfindungsgemässe Mittel als Matrix für die Laserdesorption verwendet wird. Dabei werden zunächst eine Verbindung der Formel I, Ammoniumsalz und Oligonukleotid in einem Lösungsmittel gemischt, ein Teil der Lösung auf den Probenträger aufgebracht und schliesslich das Lösungsmittel vorsichtig abgedampft. Die so auskristallisierte Probe kann dann in der matrix-unterstützen Laserdesorptions-Massenspektroskopie verwendet werden, wobei vor allem Stickstofflaser eingesetzt werden können, die eine Wellenlänge von 337 nm aussenden. Die Menge des Oligonukleotides wird bevorzugt so gewählt, dass das molare Verhältnis von Oligonukleotid zu Verbindung der Formel I $1{:}5x10^3$ bis $1{:}5x10^5$, besonders bevorzugt $1{:}10^4$ bis $1{:}10^5$ beträgt. Die erfindungsgemässen Matrices erlauben eine gute Übereinstimmung der experimentellen Molekulargewichtsbestimmung mit den errechneten Werten. Überraschenderweise sind die Verbindungen der Formel I, in denen n 2 bedeutet, besonders geeignet zur Molekulargewichtsbestimmung von Oligonukleotiden mit mehr als 25 Nukleotideinheiten und von Phosphorothioaten.

Mit Hilfe dieses Verfahrens kann nun nicht nur das Molekulargewichte eines Oligonukleotides ermittelt werden, sondern auch die exakte Abfolge seiner Nukleosidreste. Zu diesem Zweck wird das Oligonukleotid einmal gezielt vom 5'-Ende und einmal gezielt vom 3'-Ende her Nukleotid für Nukleotid abgebaut. Dazu werden Enzyme, sogenannte 5'-Exonukleasen beziehungsweise 3'-Exonukleasen, verwendet, die man entweder in Wasser oder im erforderlichen Puffer einsetzt. Besonders bevorzugt werden dabei Kalbsmilzphosphodiesterase und Schlangengiftphosphodiesterase. Aus diesem Abbau resultieren je nach Inkubationsdauer verschiedene Gemische von Oligonukleotiden unterschiedlicher Länge. Werden die Enzyme im erforderlichen Puffer eingesetzt, so entsalzt man diese Oligonukleotidgemische anschliessend nach einem dem Fachmann geläufigen Verfahren, beispielsweise durch Mikrodialyse. Danach werden die Gemische mittels matrix-unterstützter Laserdesorptions-Massenspektroskopie charakterisiert. Aus den Massendifferenzen zwischen den einzelnen Molekül-Peaks kann man auf das abgespaltene Nukleotid schliessen und so die komplette Nukleotidsequenz ermitteln. Im Vergleich mit den alternativen Sequenzierungs-Methoden der Molekularbiologie zeichnet sich das neue Verfahren einerseits durch seine Geschwindigkeit aus und andererseits werden keine toxischen oder radioaktiven Substanzen benötigt.

Darüber hinaus eröffnet dieses Verfahren neue, noch nicht bekannte Charakterisierungsmöglichkeiten für Oligonukleotide. So kann z.B. durch einfache Molekulargewichtsbestimmung eines Oligonukleotides bekannter Sequenz das Vorhandensein eines modifizierten beziehungsweise synthetischen Nukleosidrestes rasch und eindeutig nachgewiesen werden. Nach Abbau der Oligonukleotide mit 5'- oder 3'- Exonukleasen kann auch häufig die Position der modifizierten Base innerhalb des Oligonukleotides ermittelt werden, da Exonukleasen nicht-natürliche, modifizierte Nukleosidreste unter bestimmten, vom Fachmann leicht zu ermittelnden Bedingungen meist nicht abzubauen vermögen. In diesem Fall kommt der Abbau des Oligonukleotides am modifizierten Nukleosidrest zum Erliegen, was zu einem einzigen Signal des entsprechenden Oligonukleotidfragmentes im Massenspektrum führt.

Die nachfolgenden Beispiele erläutern die Erfindung näher:

## A) Herstellungsbeispiele

### Herstellung der Komposition

Beispiel A1: 5 µl einer 1 M Lösung von 2,4,6-Trihydroxyacetophenon, 5 µl einer 0,1 M Lösung von Ammoniumsulfat und 5 µl Ethanol werden mit 1 µl einer 50 µM Lösung des Oligonukleotides A mit der Basensequenz 5'-AGCTAGCT-3' gemischt. 1 µl dieser Lösung wird sodann auf einen Probenträger aufgebracht und das Lösungsmittel im Vakuum vorsichtig abgedampft. Die so vorbereitete Komposition kann nun in der matrix-unterstützten Laserdesorptions-Massenspektroskopie eingesetzt werden.

Beispiel A2: 5 µl einer 1 M Lösung von 2,4,6-Trihydroxyacetophenon, 5 µl einer 0,1 M Lösung von Ammoniumdihydrogencitrat und 5 µl Ethanol werden mit 1 µl einer 50 µM Lösung des Oligonukleotides B mit der Basensequenz 5'-d(AAT CAG GTA ACC CGC ATA GTG AAG TAT AGC TTC GAC CTA)-3' gemischt. 1 µl dieser Lösung wird sodann auf einen Probenträger aufgebracht und das Lösungsmittel im Vakuum vorsichtig abgedampft. Die so vorbereitete Komposition kann nun in der matrix-unterstützten Laserdesorptions-Massenspektroskopie eingesetzt werden.

Beispiel A3: 5 µl einer 1 M Lösung von 2,6-Dihydroxyacetophenon, 5 µl einer 0,1 M Lösung von Ammoniumdihydrogencitrat und 5 µl Ethanol werden mit 1 µl einer 50 µM Lösung des Oligonukleotides B mit der Basensequenz 5'-d(AAT CAG GTA ACC CGC ATA GTG AAG TAT AGC TTC GAC CTA)-3' gemischt. 1 µl dieser Lösung wird sodann auf einen Probenträger aufgebracht und das Lösungsmittel im Vakuum vorsichtig abgedampft. Die so vorbereitete Komposition kann nun in der matrix-unterstützten Laserdesorptions-Massenspektroskopie eingesetzt werden.

Beispiel A4: 5 µl einer 1 M Lösung von 2,6-Dihydroxyacetophenon, 5 µl einer 0,1 M Lösung von Ammoniumdihydrogencitrat und 5 µl Ethanol werden mit 1 µl einer 50 µM Lösung des Oligonukleotides C mit der Basensequenz 5'-d(TTA CGC CTA ACA GCG ATA TCA GGA CTT CAG CGT ACA GCA TTA CCA GTA TAG CCT TAG AGC)-3' gemischt. 1 µl dieser Lösung wird sodann auf einen Probenträger aufgebracht und das Lösungsmittel im Vakuum vorsichtig abgedampft. Die so vorbereitete Komposition kann nun in der matrix-unterstützten Laserdesorptions-Massenspektroskopie eingesetzt werden.

## B) Anwendungsverfahren

### Molekulargewichtsbestimmung

Beispiel B1: Phenylalanyl-tRNA aus Bierhefe wird wie im Herstellungsbeispiel A1 beschrieben zur Molekulargewichtsbestimmung vorbereitet und anschliessend mittels matrix-unterstützter Laserdesorptions-Massenspektroskopie mit einem Laser der Wellenlänge 337 nm analysiert.

Das Massenspektrum zeigt einen Hauptpeak bei 24 877,3 g/mol und einen Nebenpeak bei 12 578,0 g/mol.

Der Hauptpeak entspricht dabei dem einfach geladenen Molekülion der Phenylalanyl-tRNA, der Nebenpeak dem doppelt geladenen Molekülion.

Beispiel B2: Das Oligonukleotid B mit der Basensequenz 5'-d(AAT CAG GTA ACC CGC ATA GTG AAG TAT AGC TTC GAC CTA)-3' wird wie im Herstellungsbeispiel A2 beschrieben zur Molekulargewichtsbestimmung vorbereitet und anschliessend mittels matrix-unterstützter Laserdesorptions-Massenspektroskopie mit einem Laser der Wellenlänge 337 nm analysiert.

Das Massenspektrum zeigt einen Peak bei 12 122,3 g/mol gegenüber einer berechneten Masse von 12272,1 (Molekül minus ein Proton).

Beispiel B3: Das Oligonukleotid B mit der Basensequenz 5'-d(AAT CAG GTA ACC CGC ATA GTG AAG TAT AGC TTC GAC CTA)-3' wird wie im Herstellungsbeispiel A3 beschrieben zur Molekulargewichtsbestimmung vorbereitet und anschliessend mittels matrix-unterstützter Laserdesorptions-Massenspektroskopie mit einem Laser der Wellenlänge 337 nm analysiert.

Das Massenspektrum zeigt einen Peak bei 12 278,3 g/mol gegenüber einer berechneten Masse von 12274,1 (Molekül plus ein Proton).

Beispiel B4: Das Oligonukleotid C mit der Basensequenz 5'-d(TTA CGC CTA ACA GCG ATA TCA GGA CTT CAG CGT ACA GCA TTA CCA GTA TAG CCT TAG AGC)-3' wird wie im Herstellungsbeispiel A4 beschrieben zur Molekulargewichtsbestimmung vorbereitet und anschliessend mittels matrix-unterstützter Laserdesorptions-Massenspektroskopie mit einem Laser der Wellenlänge 337 nm analysiert.

Das Massenspektrum zeigt einen Peak bei 18 453,8 g/mol gegenüber einer berechneten Masse von 18 453,1 (Molekül plus ein Proton).

Sequenzierung

Beispiel C1: Die Sequenzierung des Oligonukleotides A (5'-AGCTAGCT-3') erfolgt durch zeitabhängigen exonukleolytischen Abbau einmal vom 5'-Ende und einmal vom 3'-Ende her.

Für den Abbau vom 5'-Ende werden 20µl einer 50µM Lösung des Oligonukleotides A mit 1µl einer Lösung des Enzyms Kalbsmilzphosphodiesterase ($2x10^{-3}$U/µl) bei 37°C inkubiert. Alle 15 Minuten wird 1ul entnommen, wie im Herstellungsbeispiel A1 beschrieben aufbereitet und dann mittels matrix-unterstützter Laserdesorptions-Massenspektroskopie charakterisiert.

Für den Abbau vom 3'-Ende werden 20µl einer 50µM Lösung des Oligonukleotides A mit 1µl einer Lösung des Enzyms Schlangengiftphosphodiesterase ($3x10^{-3}$U/µl) bei 37°C inkubiert. Alle 15 Minuten wird 1µl entnommen, wie im Herstellungsbeispiel A1 beschrieben aufbereitet und dann mittels matrix-unterstützter Laserdesorptions-Massenspektroskopie charakterisiert. Die Ergebnisse sind nachfolgend angegeben:

Oligonukleotid-Fragmente des 5'-Abbaues - Molekulargewichte in g/mol

| Molekulargewicht | Fragment |
|---|---|
| 2405,9 | 5'-AGCTANNN-3' |
| 2092,6 | 5'-GCTANNN-3' |
| 1763,4 | 5'-CTANNN-3' |
| 1474,3 | 5'-TANNN-3' |
| 1170,2 | 5'-ANNN-3' |
| 857,3 | 5'-NNN-3' |

Oligonukleotidfragmente des 3'-Abbaues - Molekulargewichte in g/mol

| Molekulargewicht | Fragment |
|---|---|
| 2405,2 | 5'-NNNTAGCT-3' |
| 2100,8 | 5'-NNNTAGC-3' |
| 1811,7 | 5'-NNNTAG-3' |
| 1482,7 | 5'-NNNTA-3' |
| 1169,6 | 5'-NNNT-3' |
| 866,0 | 5'-NNN-3' |

| | |
|---|---|
| Erwartete Sequenz: | 5'-AGCTAGCT-3' |
| Ermittelte Sequenz: | 5'-AGCTAGCT-3' |

Beispiel C2: Die Sequenzierung des Oligonukleotides D (5'-d(AAT CAG GTA ACC CGC ATA GTG AAG TAT AGC TTC G)-3' erfolgt durch zeitabhängigen exonukleolytischen Abbau einmal vom 5'-Ende und einmal vom 3'-Ende her.

Für den Abbau vom 5'-Ende werden 20 µl einer 50 µM Lösung des Oligonukleotides D mit 1 µl einer Lösung des Enzyms Kalbsmilzphosphodiesterase ($2x10^{-3}$ U/µl) bei 37°C inkubiert. Nach 3 min und nach 13 min wird 1 µl entnommen, wie im Herstellungsbeispiel A3 beschrieben aufbereitet und dann mittels matrix-unterstützter Laserdesorptions-Massenspektroskopie charakterisiert.

Für den Abbau vom 3'-Ende werden 20 µl einer 50 µM Lösung des Oligonukleotides D mit 1 µl einer Lösung des Enzyms Schlangengiftphosphodiesterase ($3x10^{-3}$U/µl) bei 37°C inkubiert. Nach 3 min, 30 min und 60 min 1 µl entnommen, wie im Herstellungsbeispiel A3 beschrieben aufbereitet und dann mittels matrix-unterstützter Laserdesorptions-Massenspektroskopie charakterisiert. Die Ergebnisse sind nachfolgend angegeben:

Oligonukleotid-Fragmente des 5'-Abbaues - Molekulargewichte in g/mol

| Molekulargewicht | Fragment |
|---|---|
| 10461,2 | 5'-ANNNNNN ....-3' |
| 10150,2 | 5'-AANNNN ....-3' |
| 9548,0 | 5'-AATNN ....-3' |

Oligonukleotidfragmente des 3'-Abbaues - Molekulargewichte in g/mol

| Molekulargewicht | Fragment |
|---|---|
| 10467,0 | 5'-NNNCAGGTAACCCGCATAGTGAAGTATAGCTTCG-3' |
| 10124,6 | 5'-NNNCAGGTAACCCGCATAGTGAAGTATAGCTTC-3' |
| 9832,5 | 5'-NNNCAGGTAACCCGCATAGTGAAGTATAGCTT-3' |
| 9530,9 | 5'-NNNCAGGTAACCCGCATAGTGAAGTATAGCT-3' |
| 9228,2 | 5'-NNNCAGGTAACCCGCATAGTGAAGTATAGC-3' |
| 8938,0 | 5'-NNNCAGGTAACCCGCATAGTGAAGTATAG-3' |

| | |
|---|---|
| 8614,1 | 5'-NNNCAGGTAACCCGCATAGTGAAGTATA-3' |
| 8299,6 | 5'-NNNCAGGTAACCCGCATAGTGAAGTAT-3' |
| 7995,8 | 5'-NNNCAGGTAACCCGCATAGTGAAGTA-3' |
| 7684,9 | 5'-NNNCAGGTAACCCGCATAGTGAAGT-3' |
| 7379,0 | 5'-NNNCAGGTAACCCGCATAGTGAAG-3' |
| 7098,0 | 5'-NNNCAGGTAACCCGCATAGTGAA-3' |
| 6783,9 | 5'-NNNCAGGTAACCCGCATAGTGA-3' |
| 6467,0 | 5'-NNNCAGGTAACCCGCATAGTG-3' |
| 6135,5 | 5'-NNNCAGGTAACCCGCATAGT-3' |
| 5829,2 | 5'-NNNCAGGTAACCCGCATAG-3' |
| 5499,4 | 5'-NNNCAGGTAACCCGCATA-3' |
| 5185,7 | 5'-NNNCAGGTAACCCGCAT-3' |
| 4879,5 | 5'-NNNCAGGTAACCCGCA-3' |
| 4564,9 | 5'-NNNCAGGTAACCCGC-3' |
| 4272,9 | 5'-NNNCAGGTAACCCG-3' |
| 3941,9 | 5'-NNNCAGGTAACCC-3' |
| 3638,1 | 5'-NNNCAGGTAACC-3' |
| 3349,1 | 5'-NNNCAGGTAAC-3' |
| 3059,8 | 5'-NNNCAGGTAA-3' |
| 2747,0 | 5'-NNNCAGGTA-3' |
| 2433,8 | 5'-NNNCAGGT-3' |
| 2129,4 | 5'-NNNCAGG-3' |
| 1800,6 | 5'-NNNCAG-3' |
| 1471,3 | 5'-NNNCA-3' |
| 1157,7 | 5'-NNNC-3' |
| 868,2 | 5'-NNN-3' |

**Patentansprüche**

1. Komposition enthaltend
   (a) mindestens ein Ammoniumsalz einer anorganischen oder organischen Säure,
   (b) eine Verbindung der Formel I,

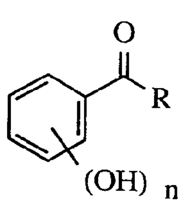

(I)

worin R einen aliphatischen Kohlenwasserstoff-Rest mit 1 bis 12 C-Atomen oder einen mit -O- Atomen

unterbrochenen aliphatischen Kohlenwasserstoff-Rest mit 2 bis 12 C-Atomen bedeutet und n eine Zahl von 1 bis 5 bedeutet und
(c) mindestens eine höhermolekulare Verbindung aus der Gruppe der Polynukleotide, Oligosaccharide, Proteine und makrozyklischen Metallkomplexe.

2. Komposition gemäss Anspruch 1, dadurch gekennzeichnet, dass Ammoniumsalz und Verbindung der Formel I zueinander in einem molaren Verhältnis von 1:5 bis 1:20 stehen.

3. Komposition gemäss Anspruch 1, dadurch gekennzeichnet, dass die höhermolekulare Verbindung und die Verbindung der Formel I zueinander in einem molaren Verhältnis von $1{:}5x10^3$ bis $1{:}5x10^5$ stehen.

4. Komposition gemäss Anspruch 1, dadurch gekennzeichnet, dass es sich bei der höhermolekularen Verbindung um ein Oligonukleotid bestehend aus 2 bis 100 Nukleosidresten handelt.

5. Komposition gemäss Anspruch 1, dadurch gekennzeichnet, dass in Formel I R für lineares oder verzweigtes Alkyl oder Cycloalkyl steht.

6. Komposition gemäss Anspruch 5, dadurch gekennzeichnet, dass in Formel I R für lineares oder verzweigtes $C_1$-$C_4$-Alkyl steht.

7. Komposition gemäss Anspruch 1, dadurch gekennzeichnet, dass sie als Verbindung der Formel I 2,4,6-Trihydroxyacetophenon, 2,4,5-Trihydroxybutyrophenon, 2,4-, 2,5- oder 2,6-Dihydroxyacetophenon enthält.

8. Komposition gemäss Anspruch 1, dadurch gekennzeichnet, dass sie mindestens ein Ammoniumsalz aus der Reihe Schwefelsäure, Salpetersäure, Tartronsäure, Aepfelsäure, Weinsäure, Zitronensäure und der Säuren HOOC-$R_1$-COOH enthält, worin $R_1$ für $C_1$-$C_8$-Alkylen, $C_5$-$C_6$-Cycloalkylen, Phenylen oder Naphthylen mit 1 bis 3 Hydroxygruppen steht.

9. Komposition gemäss Anspruch 1, dadurch gekennzeichnet, dass es sich bei der höhermolekularen Verbindung um Oligonukleotide handelt, die die natürliche Nukleosidreste Adenosin, Guanosin, Cytidin, Uridin, 2'-Desoxyadenosin, 2'Desoxythymidin, 2'-Desoxyguanosin und 2'-Desoxycytidin enthalten.

10. Komposition gemäss Anspruch 1, dadurch gekennzeichnet, dass es sich bei der höhermolekularen Verbindung um Oligonukleotide handelt, die neben den natürlichen Nukleosidresten auch Nukleosidreste enthalten, die sich von Adenin, N-Methyladenin, 2-Aminoadenin, 6-Hydroxypurin, 2-Amino-6-Chlorpurin, 2-Amino-6-methylthiopurin, Guanin, 7-Deazaguanin, N-Isobutyrylguanin, Uracil, Thymin, Cytosin, 5-Fluoruracil, 5-Chloruracil, 5-Bromuracil, Dihydrouracil und 5-Methylcytosin ableiten, sowie von deren Phosphonaten, Phosphorthioaten, Phosphoramidaten, Dephosphoderivaten und carbocyclische Derivaten.

11. Komposition gemäss Anspruch 1, dadurch gekennzeichnet, dass sie
(a) mindestens ein Ammoniumsalz aus der Reihe Schwefelsäure, Weinsäure und Zitronensäure,
(b) als Verbindung der Formel I 2,4,6-Trihydroxyacetophenon, 2,4,5-Trihydroxybutyrophenon, 2,4-, 2,5- oder 2,6-Dihydroxyacetophenon sowie
(c) mindestenes ein Oligonukleotid enthält, das aus natürlichen oder synthetischen Nukleosidresten oder einer Mischung solcher Nukleosidreste besteht.

12. Mittel zur Verwendung als Matrix in der Laserdesorption von Analytmolekülionen, dadurch gekennzeichnet, dass
(a) mindestens ein Ammoniumsalz einer anorganischen oder organischen Säure und
(b) eine Verbindung der Formel I,

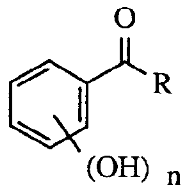

(I)

worin R einen aliphatischen Kohlenwasserstoff-Rest mit 1 bis 12 C-Atomen oder einen mit -O- Atomen unterbrochenen aliphatischen Kohlenwasserstoff-Rest mit 2 bis 12 C-Atomen und n eine Zahl von 1 bis 5 bedeutet, enthalten sind.

**13.** Mittel gemäss Anspruch 12, dadurch gekennzeichnet, dass Ammoniumsalz und Verbindung der Formel I zueinander in einem molaren Verhältnis von 1:5 bis 1:20 stehen.

**14.** Verfahren zur Analyse des Molekulargewichtes von Oligonukleotiden bestehend aus 2 bis 200 natürlichen oder synthetischen Nukleosidresten oder einer Mischung solcher Nukleosidreste, dadurch gekennzeichnet, dass man die Oligonukleotide oder deren Gemische in ein Mittel einbettet, welches
(a) mindestens ein Ammoniumsalz einer anorganischen oder organischen Säure und
(b) eine Verbindung der Formel I,

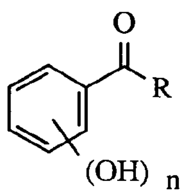

(I)

worin R einen aliphatischen Kohlenwasserstoff-Rest mit 1 bis 12 C-Atomen oder einen mit -O- Atomen unterbrochenen aliphatischen Kohlenwasserstoff-Rest mit 2 bis 12 C-Atomen und n eine Zahl von 1 bis 5 bedeutet, enthält, und sie mittels matrix-unterstützter Laserdesorptions-Massenspektroskopie charakterisiert.

**15.** Verfahren gemäss Anspruch 14, dadurch gekennzeichnet, dass die Oligonukleotide aus 2 bis 100 Nukleosidresten bestehen und relativ zur Verbindung der Formel I in einem molaren Verhältnis von $1:5x10^3$ bis $1:5x10^5$ stehen.

**16.** Verfahren zur Bestimmung der Basensequenz von Oligonukleotiden, dadurch gekennzeichnet, dass man das Oligonukleotid einmal mit einer 5'-Exonuklease und einmal mit einer 3'-Exonuklease kontrolliert abbaut, die Abbauprodukte in ein Mittel einbettet, welches
(a) mindestens ein Ammoniumsalz einer anorganischen oder organischen Säure und
(b) eine Verbindung der Formel I,

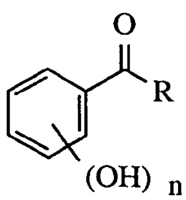

(I)

worin R einen aliphatischen Kohlenwasserstoff-Rest mit 1 bis 12 C-Atomen oder einen mit -O- Atomen unterbrochenen aliphatischen Kohlenwasserstoff-Rest mit 2 bis 12 C-Atomen und n eine Zahl von 1 bis 5 bedeutet, enthält, und sie mittels matrix-unterstützter Laserdesorptions-Massenspektroskopie analysiert.

**17.** Verfahren gemäss Anspruch 16, dadurch gekennzeichnet, dass die Oligonukleotide aus 2 bis 100

Nukleosidresten bestehen.

**18.** Verfahren gemäss Anspruch 16, dadurch gekennzeichnet, dass als 5'-Exonuklease Kalbsmilzphosphodiesterase und als 3'-Exonuklease Schlangengiftphosphodiesterase verwendet wird.

**19.** Verwendung einer Komposition gemäss den Ansprüchen 1 bis 11 zur Laserdesorption der enthaltenen höhermolekularen Verbindungen in Form von Molekülionen.